# EUROPÄISCHE PATENTSCHRIFT

(11) **EP 0 581 147 B1**
(45) Veröffentlichungstag und Bekanntmachung des Hinweises auf die Patenterteilung: **07.10.1998**
(21) Anmeldenummer: 93111462.3
(22) Anmeldetag: 16.07.1993
(51) Int. Cl.: C07D 277/36

(54) **Chlorthiazolderivate**
Chlorothiazole derivatives
Dérivés de chlorothiazols

(30) Priorität: 29.07.1992 DE 4225024
(43) Veröffentlichungstag der Anmeldung: 02.02.1994
(73) Patentinhaber: BAYER AG, 51368 Leverkusen (DE)
(72) Erfinder: Diehr, Hans-Joachim, Dr., D-42329 Wuppertal (DE)

(56) Entgegenhaltungen:
- FR-A- 2 242 386

## Beschreibung

Die Erfindung betrifft neue Chlorthiazolderivate, welche als Zwischenprodukte für Herbizide verwendet werden können, sowie Verfahren zu ihrer Herstellung.

Es ist bereits bekannt, daß bestimmte Chlorthiazolderivate, wie z.B. 2,4,5-Trichlorthiazol, zur Herstellung von herbizid wirksamen Verbindungen, wie z.B. N-Methyl-N-phenyl-α-(4,5-dichlor-1,3-thiazol-2-yl-oxy)-acetamid, verwendet werden können (vgl. EP-A 18497, Beispiel 1). Die hierbei erzielten Ausbeuten sind jedoch für industrielle Belange nicht ganz zufriedenstellend.

Weiter ist bekannt, daß Heteroaryloxyacetamide durch Umsetzung von sulfonylierten Heteroaromaten mit Oxyacetamiden hergestellt werden können (vgl. EP-A 165537). Die entsprechende Umsetzung sulfonylierter Chlorthiazole mit Oxyacetamiden ist jedoch nicht beschrieben worden.

Es wurden nun neue Chlorthiazolderivate der allgemeinen Formel (I) gefunden, 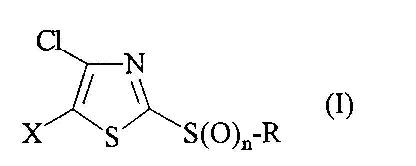 in welcher
- n: für die Zahlen 0 oder 2 steht,
- R: für Alkyl steht und
- X: für Wasserstoff oder Chlor steht.

Weiter wurde gefunden, daß man die neuen Chlorthiazolderivate der allgemeinen Formel (I) erhält, wenn man
(a) für den Fall, daß in der Formel (I) n für die Zahl 2 steht und R und X die oben angegebene Bedeutung haben,
   Chlorthiazolderivate der allgemeinen Formel (I), in welcher n für die Zahl 0 steht und R und X die oben angegebene Bedeutung haben, mit einem Oxidationsmittel, gegebenenfalls in Gegenwart eines Katalysators und gegebenenfalls in Gegenwart eines Verdünnungsmittels umsetzt,
   oder wenn man
(b) für den Fall, daß in der Formel (I) X für Chlor steht und n und R die oben angegebene Bedeutung haben,
   Thiazolderivate der allgemeinen Formel (II) 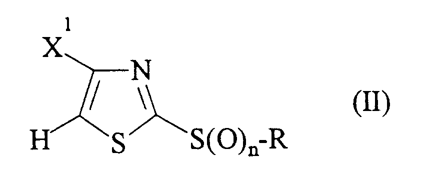 in welcher
   - n und R: die oben angegebene Bedeutung haben und
   - X¹: für Wasserstoff oder Chlor steht,
   mit Chlor, gegebenenfalls in Gegenwart eines Katalysators und gegebenenfalls in Gegenwart eines Verdünnungsmittels umsetzt.

Überraschenderweise können durch Umsetzung der neuen Chlorthiazolderivate der Formel (I) mit Hydroxyacetamiden die entsprechenden Chlorthiazolyloxyacetamide in nahezu der theoretischen Ausbeute erhalten werden, wahrend dies nach der bisher bekannten Verfahrensweise unter Verwendung von 2,4,5-Trichlor-thiazol nicht gelungen war.

Das erfindungsgemäße Verfahren stellt somit eine wertvolle Bereicherung des Standes der Technik dar.

Die Erfindung betrifft vorzugsweise neue Chlorthiazolderivate der Formel (I), in welcher
- n: für die Zahlen 0 oder 2 steht,
- R: für geradkettiges oder verzweigtes Alkyl mit 1 bis 4 Kohlenstoffatomen steht und
- X: für Wasserstoff oder Chlor steht.

Die Erfindung betrifft insbesondere Verbindungen der Formel (I), in welcher
- n: für die Zahlen 0 oder 2 steht,
- R: für Methyl oder Ethyl steht und
- X: für Wasserstoff oder Chlor steht.

Als Beispiele für die Verbindungen der Formel (I) seien genannt:
4-Chlor-2-methylthio-thiazol, 4-Chlor-2-methylsulfonyl-thiazol, 4,5-Dichlor-2-methylthio-thiazol und 4,5-Dichlor-2-methylsulfonyl-thiazol.

Verwendet man beispielsweise 4,5-Dichlor-2-methylthio-thiazol und Hydrogenperoxid als Ausgangsstoffe, so kann der Reaktionsablauf beim erfindungsgemäßen Verfahren (a) durch das folgende Formelschema skizziert werden:

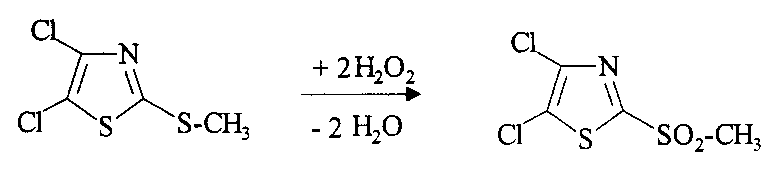

Verwendet man beispielsweise 4-Chlor-5-methylthio-thiazol und Chlor als Ausgangsstoffe, so kann der Reaktionsablauf beim erfindungsgemäßen Verfahren (b) durch das folgende Formelschema skizziert werden:

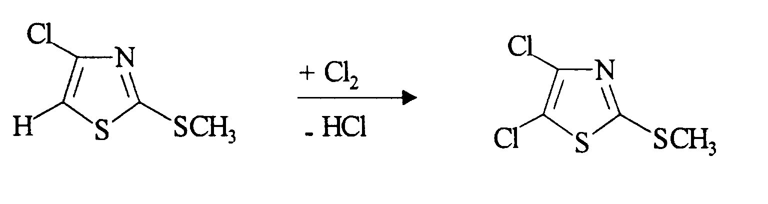

Die beim erfindungsgemäßen Verfahren (a) als Ausgangsstoffe benötigten Verbindungen der Formel (I) sind erfindungsgemäß beanspruchte neue Verbindungen. Sie können nach dem erfindungsgemäßen Verfahren (b) hergestellt werden.

Das erfindungsgemäße Verfahren (a) wird unter Verwendung eines Oxidationsmittels durchgeführt. Für den Einsatz beim erfindungsgemäßen Verfahren (a) geeignete Oxidationsmittel sind vorzugsweise Hydrogenperoxid (H₂O₂), Persäuren oder deren Salze, wie Perameisensäure, Peressigsäure, Perpropionsäure, Perbenzoesäure, 3-Chlorperbenzoesäure, Kaliumhydrogenpersulfat, Kaliumperoxodisulfat oder Natriumperoxodisulfat, ferner auch hypochlorige Säure oder deren Metallsalzlösungen in Wasser.

Das erfindungsgemäße Verfahren (a) wird gegebenenfalls unter Verwendung eines Katalysators durchgeführt. Herbei sind als Katalysatoren vorzugsweise Salze von Metallen der IV., V. und VI. Nebengruppe des Periodensystems der Elemente geeignet. Als Beispiele hierfür seien Natrium(meta)vanadat, Natriummolybdat und Natriumwolframat genannt.

Das erfindungsgemäße Verfahren (a) wird vorzugsweise unter Verwendung eines Verdünnungsmittels durchgeführt. Als Verdünnungsmittel kommen dabei neben Wasser die für Oxidätionsreaktionen üblichen organischen Solventien in Betracht. Hierzu gehören vorzugsweise chlorierte Kohlenwasserstoffe, wie Methylenchlorid, Ethylenchlorid, Chloroform, Tetrachlormethan, 1,1,2-Trichlorethan, Chlorbenzol und o-Dichlorbenzol, Alkohole, wie Methanol, Ethanol, Propanol, Isopropanol, Butanol, Isobutanol und sec-Butanol, Carbonsäuren, wie Ameisensäure, Essigsäure und Propionsäure.

Die Reaktionstemperaturen können bei dem erfindungsgemäßen Verfahren (a) in einem größeren Bereich variiert werden. Im allgemeinen arbeitet man bei Temperaturen zwischen -20°C und +120°C, vorzugsweise bei Temperaturen zwischen 0°C und 80°C.

Das erfindungsgemäße Verfahren (a) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (a) setzt man auf 1 Mol Ausgangsverbindung der Formel (I) im allgemeinen zwischen 1 und 10 Mol, vorzugsweise zwischen 1 und 5 Mol Oxidationsmittel, und gegebenenfalls zwischen 0,001 und 0,1 Mol, vorzugsweise zwischen 0,01 und 0,05 Mol eines Katalysators ein.

In einer bevorzugten Ausführungsform des erfindungsgemäßen Verfahrens (a) wird die Ausgangsverbindung der Formel (I) und gegebenenfalls ein Katalysator in einem Verdünnungsmittel vorgelegt und bei Raumtemperatur wird unter Rühren das Oxidationsmittel eindosiert. Dann wird das Reaktionsgemisch, gegebenenfalls bei erhöhter Temperatur, bis zum Ende der Umsetzung gerührt. Die Aufarbeitung kann anschließend auf übliche Weise durchgeführt werden (vgl. die Herstellungsbeispiele).

Die beim erfindungsgemäßen Verfahren (b) zur Herstellung von Verbindungen der Formel (I) als Ausgangsstoffe zu verwendenden Thiazolderivate sind durch die Formel (II) allgemein definiert.

In Formel (II) haben n und R vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n und R angegeben wurden.

Die Verbindungen der Formel (II), in welcher X¹ für Wasserstoff steht, sind bekannt und/oder können nach an sich bekannten Verfahren hergestellt werden (vgl. Synthesis 1985, 948-949; J. Het. Chem. 15 (1978), 1361-1366).

Die Verbindungen der Formel (II), in welcher X¹ für Chlor steht, sind noch nicht aus der Literatur bekannt und als neue Stoffe ebenfalls Gegenstand der vorliegenden Patentanmeldung.

Man erhält die Verbindungen der Formel (II), in welcher X¹ für Chlor steht, wenn man Thiazolinone der allgemeinen Formel (III) 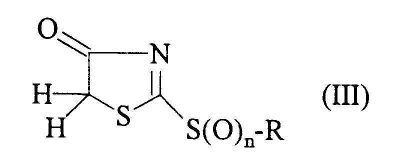 in welcher
- n und R: die oben angegebene Bedeutung haben,
mit einem Säurechlorid, wie z.B. Phosphorylchlorid (POCl₃), gegebenenfalls in Gegenwart eines Reaktionshilfsmittels, wie z.B. Pyridin, bei Temperaturen zwischen 0°C und 100°C umsetzt und auf übliche Weise aufarbeitet (vgl. die Herstellungsbeispiele).

Die Thiazolinone der Formel (III) sind bekannt und/oder können nach an sich bekannten Verfahren hergestellt werden (vgl. Bull. Soc. Chim. France 1968, 3461-3468).

Das erfindungsgemäße Verfahren (b) wird vorzugsweise in Gegenwart eines Katalysators durchgeführt. Als Katalysatoren kommen vorzugsweise Verbindungen in Betracht, welche als Radikalstarter für chemische Reaktionen eingegesetzt werden können. Hierzu gehören insbesondere Peroxide, wie Benzoylperoxid, und Azoverbindungen, wie Azo-bis-isobutyronitril.

Das erfindungsgemäße Verfahren (b) wird vorzugsweise unter Verwendung eines Verdünnungsmittels durchgeführt. Als Verdünnungsmittel kommen die für Chlorierungsreaktionen üblichen organischen Solventien in Betracht. Hierzu gehören insbesondere chlorierte Kohlenwasserstoffe, wie Methylenchlorid, Chloroform, Tetrachlormethan, Ethylenchlorid, 1,1,2-Trichlorethan, Chlorbenzol und o-Dichlorbenzol.

Die Reaktionstemperaturen können bei dem erfindungsgemäßen Verfahren (b) in einem größeren Bereich variiert werden. Im allgemeinen arbeitet man bei Temperaturen zwischen 0°C und 150°C, vorzugsweise bei Temperaturen zwischen 20°C und 120°C.

Das erfindungsgemäße Verfahren (b) wird im allgemeinen unter Normaldruck durchgeführt. Es ist jedoch auch möglich, unter erhöhtem oder vermindertem Druck - im allgemeinen zwischen 0,1 und 10 bar - zu arbeiten.

Zur Durchführung des erfindungsgemäßen Verfahrens (b) setzt man auf 1 Mol Ausgangsverbindung der Formel (II) im allgemeinen zwischen 1 und 5 Mol, vorzugsweise zwischen 1 und 3 Mol Chlor und gegebenenfalls zwischen 0,001 und 0,1 Mol, vorzugsweise zwischen 0,01 und 0,05 Mol eines Katalysators ein.

In einer bevorzugten Ausführungsform des erfindungsgemäßen Verfahrens (b) wird die Ausgangsverbindung der Formel (II) mit einem Verdünnungsmittel und einem Katalysator bei Raumtemperatur vermischt, die Mischung wird auf die erforderliche Reaktionstemperatur - vorzugsweise zwischen 60°C und 120°C - aufgeheizt und dann wird bei dieser Temperatur Chlor bis zum Ende der Umsetzung eingeleitet. Anschließend wird aufübliche Weise aufgearbeitet (vgl. die Herstellungsbeispiele).

Die neuen Chlorthiazolderivate der Formel (I) können zur Herstellung von herbizid wirksamen Heteroaryloxyacetamiden verwendet werden. Verwendet man beispielsweise 4,5-Dichlor-2-methylsulfonyl-thiazol und N-Methyl-N-phenyl-α-hydroxyacetamid als Komponenten für diese Umsetzung, so kann die ablaufende Reaktion zur Herstellung von N-Methyl-N-phenyl-α-(4,5-dichlor-1,3-thiazol-2-yl-oxy)-acetamid (vgl. EP-A 18497) durch das folgende Formelschema skizziert werden:

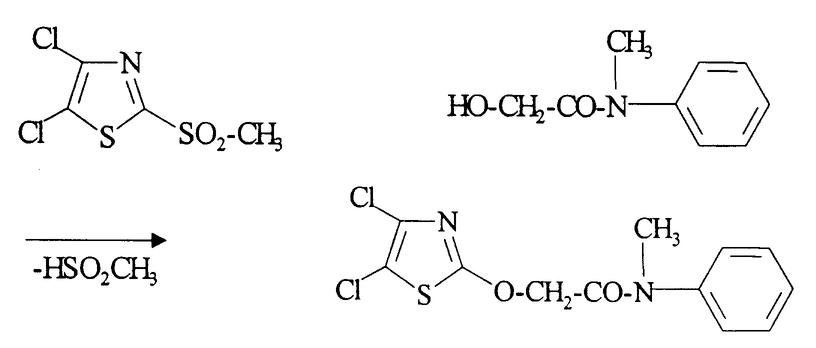

### Herstellungsbeispiele:

### Beispiel 1

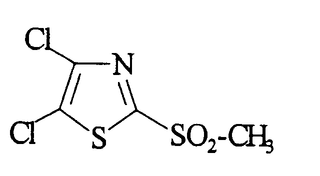

### (Verfahren (a))

15,0 g (0,07 Mol) 4,5-Dichlor-2-methylthio-thiazol werden in 100 ml Essigsäure gelöst und mit einer Spatelspitze Natriumwolframat versetzt. Zu dieser Lösung werden bei 20°C unter Rühren 30 ml einer 35%igen wäßrigen Hydrogenperoxid-Lösung (0,35 Mol H₂O₂) tropfenweise gegeben. Das Reaktionsgemisch wird dann 60 Minuten bei 60°C bis 70°C gerührt und mit Eiswasser auf etwa das dreifache Volumen verdünnt. Das hierbei kristallin angefallene Produkt wird durch Absaugen isoliert.

Man erhält 14,5 g (89% der Theorie) 4,5-Dichlor-2-methylsulfonyl-thiazol vom Schmelzpunkt 106°C.

### Beispiel 2

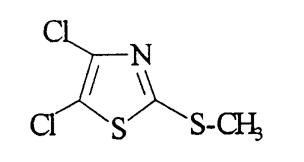

### (Verfahren (b))

66 g (0,40 Mol) 4-Chlor-2-methylthio-thiazol werden in 150 ml Chlorbenzol gelöst und mit einer Spatelspitze Azo-bis-isobutyronitril (2,2'-Azoisobuttersäurenitril) versetzt. Die Mischung wird dann auf 80°C bis 90°C aufgeheizt und bei dieser Temperatur wird Chlor bis zum Ende der Umsetzung eingeleitet (ca. 90 Minuten). Das Lösungsmittel wird dann im Wasserstrahlvakuum abdestilliert und der Rückstand im Ölpumpenvakuum destilliert.

Man erhält 60,7 g (76% der Theorie) 4,5-Dichlor-2-methylthio-thiazol vom Siedepunkt 95°C bis 96°C (bei 1hPa)
Schmelzpunkt: 35°C.

### Beispiel 3

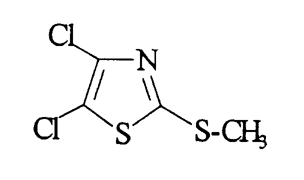

### (Verfahren (b))

In eine Mischung aus 13,1 g (0,10 Mol) 2-Methylthio-thiazol, 100 ml Chlorbenzol und einer Spatelspitze Azo-bis-isobutyronitril wird bei 100°C bis 105°C Chlor bis zum Ende der Umsetzung eingeleitet (ca. 4 Stunden). Das Reaktionsprodukt wird durch Vakuumdestillation isoliert.

Man erhält 12,3 g (61% der Theorie) 4,5-Dichlor-2-methylthio-thiazol vom Siedepunkt 95°C bis 96°C (bei 1hPa)
Schmelzpunkt: 35°C.

### Ausgangsstoffe der Formel (II)

### Beispiel (II-1)

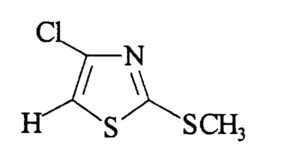

Eine Mischung aus 73,5 g (0,50 Mol) 2-Methylthio-thiazolin-(5H)-4-on [Verbindung (III), R = CH₃, n = 0], 180 ml (1,96 Mol) Phosphorylchlorid und 6 ml Pyridin wird langsam auf 90°C aufgeheizt und bis zum Ende der Gasentwicklung (ca. 60 Minuten) bei dieser Temperatur gerührt. Dann wird eingeengt, der Rückstand in Chloroform aufgenommen, die Lösung mit Eiswasser gewaschen, mit Natriumsulfat getrocknet und filtriert. Vom Filtrat wird das Lösungsmittel im Wasserstrahlvakuum sorfältig abdestilliert.

Man erhält 66,4 g (80,5% der Theorie) 4-Chlor-2-methylthio-thiazol als kristallinen Rückstand vom Schmelzpunkt 30°C.

### Verwendungsbeispiel:

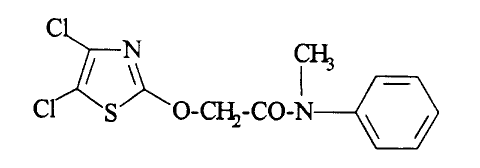

Eine Lösung von 2,4 g (0,06 Mol) Natriumhydroxid (Plätzchen) in 10 ml Wasser wird bei 20°C zu einer Mischung aus 11,6 g (0,05 Mol) 4,5-Dichlor-2-methylsulfonyl-thiazol, 8,3 g (0,05 Mol) N-Methyl-N-phenyl-α-hydroxyacetamid und 50 ml Aceton unter Rühren tropfenweise gegeben.

Die Reaktionsmischung wird 60 Minuten bei 20°C bis 25°C gerührt, dann auf 125 ml Eiswasser gegossen und mit konzentrierter Salzsäure auf pH5 eingestellt. Das hierbei kristallin angefallene Produkt wird durch Absaugen isoliert.

Man erhält 14,6 g (92% der Theorie) N-Methyl-N-phenyl-a-(4,5-dichlor-1,3-thiazol-2-yl-oxy)-acetamid vom Schmelzpunkt 88°C.

## Patentansprüche

1. Chlorthiazolderivate der allgemeinen Formel (I), 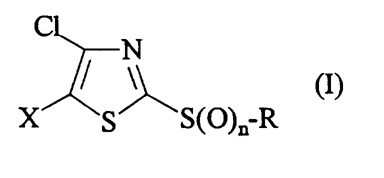 in welcher
n für die Zahlen 0 oder 2 steht,
R für Alkyl steht und
X für Wasserstoff oder Chlor steht.

2. Chlorthiazolderivate der Formel (I) gemäß Anspruch 1, dadurch gekennzeichnet, daß darin
n für die Zahlen 0 oder 2 steht,
R für geradkettiges oder verzweigtes Alkyl mit 1 bis 4 Kohlenstoffatomen steht und
X für Wasserstoff oder Chlor steht.

3. Chlorthiazolderivate der Formel (I) gemäß Anspruch 1, dadurch gekennzeichnet, daß darin
n für die Zahlen 0 oder 2 steht,
R für Methyl oder Ethyl steht und
X für Wasserstoff oder Chlor steht.

4. Verfahren zur Herstellung von Chlorthiazolderivaten der Formel (I) gemäß Anspruch 1, dadurch gekennzeichnet, daß man
(a) für den Fall, daß in der Formel (I) n für die Zahl 2 steht und R und X die oben angegebene Bedeutung haben,
Chlorthiazolderivate der allgemeinen Formel (I), in welcher n für die Zahl 0 steht und R und X die oben angegebene Bedeutung haben, mit einem Oxidationsmittel, gegebenenfalls in Gegenwart eines Katalysators und gegebenenfalls in Gegenwart eines Verdünnungsmittels umsetzt,
oder daß man
(b) tur den Fall, daß in der Formel (I) X für Chlor steht und n und R die oben angegebene Bedeutung haben,
Thiazolderivate der allgemeinen Formel (II), 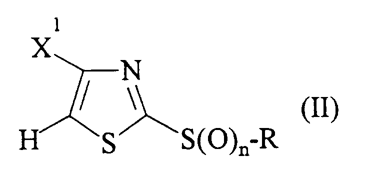 in welcher
n und R die oben angegebene Bedeutung haben und
X¹ für Wasserstoff oder Chlor steht,
mit Chlor, gegebenenfalls in Gegenwart eines Katalysators und gegebenenfalls in Gegenwart eines Verdünnungsmittels umsetzt.

## Claims

1. Chlorothiazole derivatives of the general formula (I) 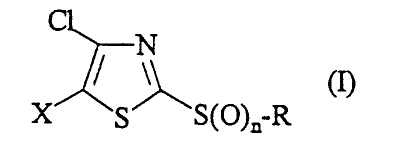 in which
n represents the numbers 0 or 2,
R represents alkyl and
X represents hydrogen or chlorine.

2. Chlorothiazole derivatives of the formula (I) according to Claim 1, characterised in that therein
n represents the numbers 0 or 2,
R represents straight-chain or branched alkyl having 1 to 4 carbon atoms and
X represents hydrogen or chlorine.

3. Chlorothiazole derivatives of the formula (I) according to Claim 1, characterised in that therein
n represents the numbers 0 or 2,
R represents methyl or ethyl and
X represents hydrogen or chlorine.

4. Process for preparing chlorothiazole derivatives of the formula (I) according to Claim 1, characterised in that
(a) in the case where in the formula (I) n represents the number 2 and R and X have the abovementioned meaning,
chlorothiazole derivatives of the general formula (I) in which n represents the number 0 and R and X have the abovementioned meaning are reacted with an oxidising agent, optionally in the presence of a catalyst and optionally in the presence of a diluent,
or in that
(b) in the case where in the formula (I) X represents chlorine and n and R have the abovementioned meaning,
thiazole derivatives of the general formula (II) 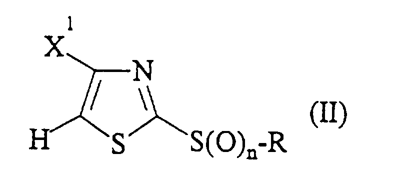 in which
n and R have the abovementioned meaning and
X¹ represents hydrogen or chlorine,
are reacted with chlorine, optionally in the presence of a catalyst and optionally in the presence of a diluent.

## Revendications

1. Dérivés de chlorothiazole de formule générale (I), 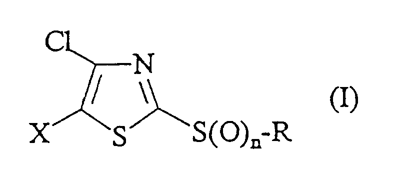 dans laquelle
n représente les nombres 0 ou 2,
R représente un alkyle, et
X représente l'hydrogène ou le chlore.

2. Dérivés de chlorothiazole de formule générale (I) selon la revendication 1, caractérisés en ce que
n représente les nombres 0 ou 2,
R représente un alkyle linéaire ou ramifié comptant de 1 à 4 atomes de carbone, et
X représente l'hydrogène ou le chlore.

3. Dérivés de chlorothiazole de formule générale (I) selon la revendication 1, caractérisés en ce que
n représente les nombres 0 ou 2,
R représente le méthyle ou l'éthyle, et
X représente l'hydrogène ou le chlore.

4. Procédé de préparation de dérivés de chlorothiazole de formule (I) selon la revendication 1, caractérisés en ce que
(a) au cas où dans la formule (I), n représente le nombre 2 et R et X ont la signification donnée plus haut,
on fait réagir avec un agent d'oxydation, éventuellement en présence d'un catalyseur et éventuellement en présence d'un agent de dilution, des dérivés du chlorothiazole de la formule générale (I) dans laquelle n représente le nombre 0 et R et X ont la signification donnée plus haut,
ou lorsque
(b) au cas où dans la formule (I), X représente le chlore et n et R ont la signification donnée plus haut,
on fait réagir des dérivés de thiazole de formule générale (II) 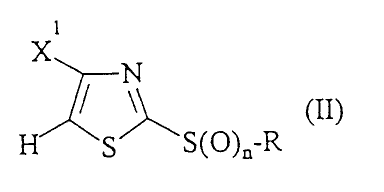 dans laquelle
n et R ont la signification donnée plus haut et
X¹ représente l'hydrogène ou le chlore,
avec du chlore, éventuellement en présence d'un catalyseur et éventuellement en présence d'un agent de dilution.
